# FASCICULE DE BREVET EUROPEEN

(11) **EP 1 623 701 B1**
(45) Date de publication et mention de la délivrance du brevet: **14.02.2007**
(21) Numéro de dépôt: 05291180.7
(22) Date de dépôt: 01.06.2005
(51) Int. Cl.: A61K 8/41, A61Q 5/06

(54) **Utilisation de dérivés de triarylméthane à titre de colorant direct dans une composition tinctoriale, composition et procédé de mise en oeuvre.**
Verwendung von Triarylmethan-Derivaten als direkte Farbstoffe in Färbemittel, Zusammensetzung und Verfahren zur deren Verwendung
Use of triarylmethane derivatives in colourant compositions, compositions and their methods of use.

(30) Priorité: 01.06.2004 FR 0405888
(43) Date de publication de la demande: 08.02.2006
(73) Titulaire: L'ORÉAL, 75008 Paris (FR)
(72) Inventeur: Lagrange Alain, 77700 Coupvray (FR); Guerin Frédéric, 75009 Paris (FR)
(74) Mandataire: Bulle, Françoise

(56) Documents cités:
- WO-A-00/64986
- WO-A-03/007296

## Description

La présente demande a pour objet l'utilisation de dérivés de triarylméthane à titre de colorant direct dans une composition tinctoriale pour fibres kératiniques, en particulier les fibres kératiniques humaines, telles que les cheveux.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs, tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques par une coloration directe ou semi-permanente. Le procédé classiquement utilisé en coloration directe consiste à appliquer sur les fibres kératiniques des colorants directs qui sont des molécules colorées et colorantes ayant une affinité pour les fibres, à laisser pauser pour permettre aux molécules colorées de pénétrer, par diffusion, à l'intérieur du cheveu, puis à rincer les fibres.

Contrairement aux compositions de teinture par oxydation, les compositions de teinture directes ou semi-permanentes sont mises en oeuvre sans la présence obligatoire d'un agent oxydant. Ces teintures peuvent être effectuées de manière répétée sans dégrader la fibre kératinique.

Il est connu par exemple d'utiliser des colorants directs nitrés benzèniques, anthraquinoniques, nitropyridiniques, azoïques, xanthéniques, acridiniques, aziniques ou triarylméthaniques.

Il en résulte des colorations souvent chromatiques qui sont cependant temporaires ou semi-permanentes à cause de la nature des liaisons entre les colorants directs et la fibre kératinique. Ces interactions font que la désorption des colorants de la surface et/ou du coeur de la fibre se fait facilement. Les colorations présentent généralement une faible puissance tinctoriale et une mauvaise tenue aux lavages ou à la transpiration.

Il existe un réel besoin de disposer de compositions de teinture directe améliorées en termes d'innocuité, de ténacité et de sélectivité, cette dernière résultant de la différence de montée entre différentes parties d'un cheveu ou d'une chevelure.

De manière surprenante et avantageuse, la Demanderesse vient de découvrir que l'utilisation de dérivés de triarylméthane à titre de colorants directs dans des compositions pour la teinture des fibres kératiniques, en particulier des fibres kératiniques humaines, telles que les cheveux, permet ces améliorations.

Outre leur avantage en terme d'innocuité, les compositions comprenant ces dérivés de triarylméthane particuliers conduisent à des teintures résistantes aux agents extérieurs (soleil, intempéries) ainsi qu'aux shampooings et à la transpiration.

En outre, ces compositions présentent un bon profil toxicologique.

Un premier objet de la présente demande est l'utilisation de dérivés de triarylméthane particuliers à titre de colorants directs dans une composition tinctoriale pour fibres kératiniques, en particulier les fibres kératiniques humaines, telles que les cheveux, ou pour la fabrication de telles compositions.

Un second objet de la présente demande est une composition tinctoriale aqueuse pour la teinture des fibres kératiniques, en particulier des fibres kératiniques humaines, telles que les cheveux comprenant dans un milieu de teinture approprié au moins un colorant direct dérivé de triarylméthane particulier, au moins une base d'oxydation et/ou au moins un colorant direct différent du dérivé de triarylméthane particulier.

Un troisième objet de la présente demande est un procédé de teinture des fibres kératiniques, en particulier des fibres kératiniques humaines, telles que les cheveux mettant en oeuvre la composition tinctoriale selon l'invention.

Un autre objet de la présente demande est un procédé d'éclaircissement des fibres kératiniques, en particulier des fibres kératiniques humaines, telles que les cheveux mettant en oeuvre la composition tinctoriale selon l'invention.

Un autre objet de la présente demande est l'utilisation de ladite composition pour la teinture des fibres kératiniques, en particulier des fibres kératiniques humaines, telles que les cheveux.

Un autre objet de la présente demande est un dispositif à compartiment ou « kit » comprenant la composition tinctoriale selon la présente invention.

Un autre objet de la présente invention est une composition tinctoriale aqueuse pour la teinture des fibres kératiniques humaines, telles que les cheveux, comprenant dans un milieu approprié au moins un composé leuco, qui est un précurseur du colorant direct dérivé de triarylméthane particulier, au moins une base d'oxydation et/ou au moins un colorant direct différent du dérivé de triarylméthane particulier.

Un autre objet de la présente demande est un procédé de coloration des fibres kératiniques mettant en oeuvre la composition comprenant le composé leuco, précurseur du colorant direct dérivé de triarylméthane particulier.

Enfin un autre objet de la présente invention est un dispositif à compartiment ou « kit » comprenant la composition comprenant le composé leuco, précurseur du colorant direct dérivé de triarylméthane particulier.

D'autres caractéristiques, aspects, objets et avantages de la présente invention apparaîtront encore plus clairement à la lecture de la description et de l'exemple qui suit.

Les colorants directs dérivés de triarylméthane utilisables dans les compositions selon la présente invention, correspondent au composé de formule (Ia) suivante : 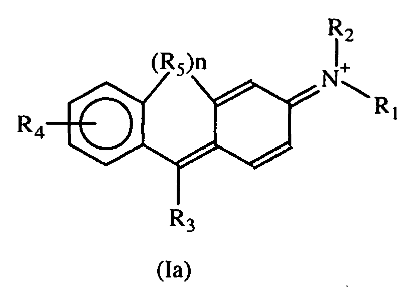 dans lesquelles :
R₁, R₂ et R₃ représentent, indépendamment les uns des autres, un atome d'hydrogène, un radical alkyle en C₁-C₂₄, alcényle ou alcynyle en C₂-C₂₄, linéaire ou ramifié, substitué ou non substitué, un radical benzyle substitué ou non substitué ou un radical aryle substitué ou non substitué, un radical hétérocycle aromatique ou non, substitué ou non,
R₄ représente un atome d'hydrogène ou un radical amino substitué ou non substitué,
R₅ représente un groupement -CR₆R₇- dans lequel R₆ et R₇ représentent, indépendamment l'un de l'autre, un atome d'hydrogène, un radical alkyle en C₁-C₂₄, linéaire ou ramifié, substitué ou non substitué, pouvant se cycliser, un radical benzyle substitué ou non substitué ou un radical aryle substitué ou non substitué,
n désigne 0 ou 1,
le dérivé de formule (Ia) pouvant éventuellement porter au moins un autre substituant pouvant être, de préférence, choisi parmi un radical alkyle en C₁-C₆ linéaire ou ramifié, un radical alcoxy en C₁-C₆ linéaire ou ramifié, un radical aryle, un atome d'halogène, notamment le chlore, ou un groupement nitro.

L'invention couvre également les formes mésomères des composés de formule (Ia).

Dans le cadre des définitions des groupes R₁, R₂, R₃, R₆ et/ou R₇ par « substitué », on entend substitué par le ou les substituants pouvant être choisis parmi un groupement alkyle en C₁-C₆ linéaire ou ramifié excepté lorsque les groupes R₁, R₂, R₃, R₆ et/ou R₇ portant le substituant représentent des groupements alkyle, alcényle ou alcynyle ; un groupement hydroxyalkyle en C₁-C₆ linéaire ou ramifié excepté lorsque les groupes R₁, R₂, R₃, R₆ et/ou R₇ portant le substituant représentent des groupements alkyle, alcényle ou alcynyle ; un groupement acétylamino ; un groupement alkylcarbonyle en C₁-C₆ linéaire ou ramifié ; un groupement alcoxy en C₁-C₆ linéaire ou ramifié ; un groupement hydroxycarbonyle ; un groupement alcoxycarbonyle en C₁-C₆ linéaire ou ramifié ; un groupement hydroxy ; un groupement amino ; un groupement mono ou dialkylamino ; un groupement mono ou dihydroxyalkylamino, un groupement nitro, un atome d'halogène ; un groupement aryle, un hétérocycle aromatique ou non, ces groupements cycliques étant non substitués ou substitués par les substituants précédemment cités.
De préférence, lorsque ces groupements cycliques sont substitués, le ou les substituants sont notamment choisis parmi un groupement hydroxy, un groupement alcoxy en C₁-C₆, un groupement amino, un groupement nitro et/ou un atome d'halogène.
De préférence, lorsque ces groupements cycliques représentent des hétérocycles aromatiques ou non, ils ne sont pas substitués.

Dans le cas où R₃ représente un radical aryle substitué ou non substitué, le radical aryle peut être condensé ou non. Le radical aryle peut ainsi représenter un radical napthalène.

Dans le cas où R₃ représente un hétérocycle aromatique ou non, substitué ou non substitué, l'hétérocycle peut être condensé on non pouvant ainsi représenter un radical indole.

L'électroneutralité du composé de formule (Ia) peut être assurée par un contre-ion Y dont la nature chimique n'est pas critique.

Le contre-ion Y peut être choisi parmi un anion ou un mélange d'anions cosmétiquement acceptables. De préférence, le contre-ion Y est choisi parmi les halogénures tels que les chlorures, les bromures, les fluorures ou les iodures ; les hydroxydes ; les sulfates ; les hydrogénosulfates ; les alkyl(C₁-C₆)sulfates ; les phosphates ; les carbonates ; les hydrogénocarbonates ; les perchlorates ; les acétates ; les tartrates ; les citrates, les oxalates ; les alkyl(C₁-C₆)sulfonates tels que le méthylsulfonate ; les arylsulfonates substitués ou non par un radical alkyle en C₁-C₄ tels que par exemple un 4-toluylsulfonate.

De manière préférée, les composés de formule (Ia) utilisables dans les compositions selon la présente invention sont tels que R₁ et R₂ représentent, indépendamment l'un de l'autre, un atome d'hydrogène, un radical alkyle en C₁-C₆, un radical mono ou polyhydroxyalkyle en C₁-C₆.

De manière préférée, les composés de formule (Ia) utilisables dans les compositions selon la présente invention sont tels que R₃ représente un atome d'hydrogène ou un radical aryle éventuellement substitué par un groupement amino, dialkylamino ou dihydroxyalkyle amino.

De manière préférée, n désigne 1 et R₅ désigne de préférence le groupement divalent -CR₆R₇-, R₆ et R₇ désignent un alkyle en C₁-C₄, éventuellement substitué par un ou plusieurs atomes d'halogène.

De préférence, les composés de formule (Ia) comprennent 2 autres substituants portés sur le tricycle.

A titre d'exemple de composés de formule (Ia) utilisables selon l'invention, on peut citer les composés suivants :

| Structure | Nom du colorant |
|---|---|
| 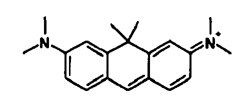 | (7-Diméthylamino-9,9-diméthyl-9H-anthracen-2-ylidène)-diméthyl-ammonium |
| 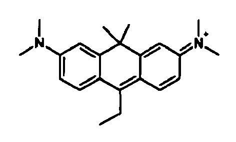 | [7-Diméthylamino-10-éthyl-9,9-diméthyl-9H-anthracen-2-ylidèn]-diméthyl ammonium |
| 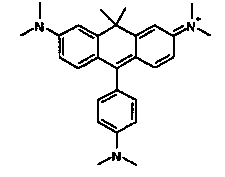 | [7-Diméthylamino-10-(4-diméthylamino-phényl)-9,9-diméthyl-9H-anthracen-2-ylidèn]- diméthyl ammonium |
| 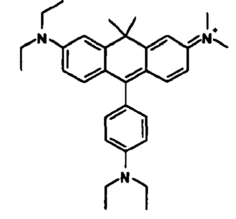 | [7-Diéthylamino-10-(4-diéthylamino-phényl)-9,9-diméthyl-9H-anthracen-2-ylidène]- diméthyl-ammonium |
| 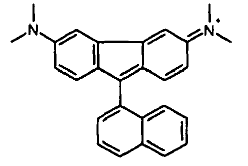 | Méthanaminium, N-[6-(diméthylamino)-9-(1-naphthalenyl)-3H-fluoren-3-ylidène]-N-méthyle |
| 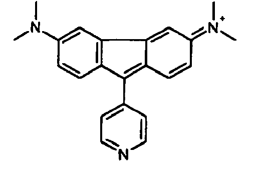 | Méthanaminium, N-[6-(diméthylamino)-9-(4-pyridinyl)-3H-fluoren-3-ylidène]-N-méthyle |
| 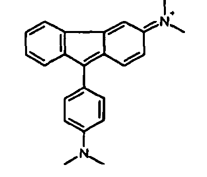 | Méthanaminium, N-[9-[4-(diméthylamino)phenyl]-3H-fluoren-3-ylidène]-N-méthyle |
| 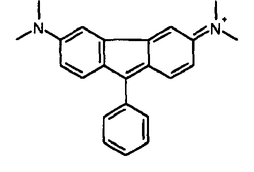 | Bis[3,6-bis(diméthylamino)-9-phénylfluoren-9-ylium] |
| 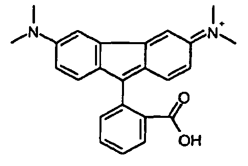 | Acide benzoïque, 2-[3,6-bis(diméthylamino)-9H-fluoren-9-ylium] |
| 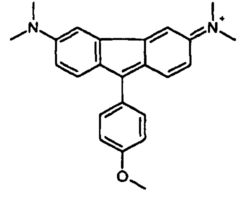 | 3,6-Bis(diméthylamino)-9-(p-méthoxyphényl)fluoren-9-ylium |
| 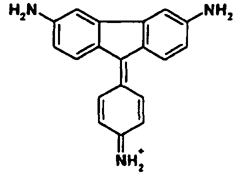 | 4-(3,6-Diamino-fluoren-9-ylidène)-cyclohexa-2,5-diénylidène-ammonium |
| 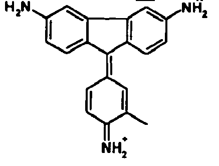 | [4-(3,6-Bis-amino-fluoren-9-ylidène)-2-méthyl-cyclohexa-2,5-diénylidene]-ammonium |
| 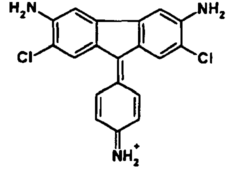 | [4-(3,6-Bis-amino-2,7-dichloro-fluoren-9-ylidène)-cyclohexa-2,5-diénylidène]-ammonium |
| 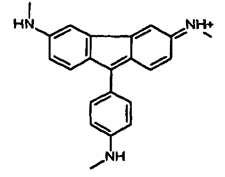 | 9H-Fluorène-3,6-diamine, N,N'-diméthyl-9-[4-(méthylamino)phényl] |
| 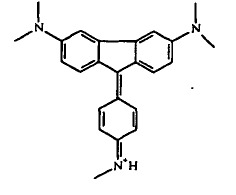 | [4-(3,6-Bis-diméthylamino-fluoren-9-ylidène)-cyclohexa-2,5-diénylidène]-méthyl-ammonium |
| 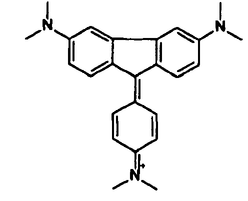 | [4-(3,6-Bis-diméthylamino-fluoren-9-ylidène)-cyclohexa-2,5-diénylidène]-diméthyl-ammonium |
| 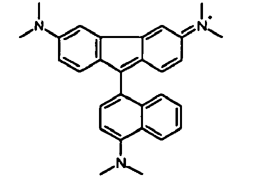 | Méthanaminium, N-[6-(diméthylamino)-9-[4-(diméthylamino)-1-naphthalényl]-3H-fluoren-3-ylidène]-N-méthyle |
| 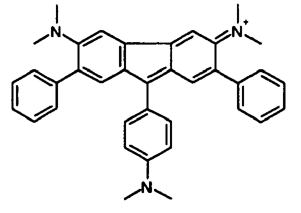 | Méthanaminium, N-[6-(diméthylamino)-9-[4-(diméthylamino)phényl]-2,7-diphényl-3H-fluoren-3-ylidène]-N-méthyle |
| 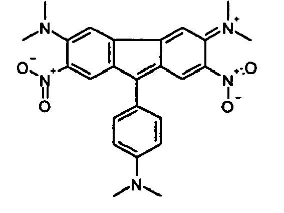 | Méthanaminium, N-[6-(diméthylamino)-9-[4-(diméthylamino)phényl]-2,7-dinitro-3H-fluoren-3-ylidène]-N-méthyle |
| 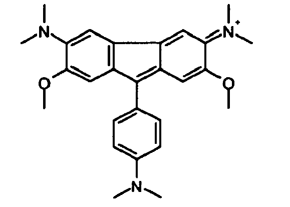 | Méthanaminium, N-[6-(diméthylamino)-9-[4-(diméthylamino)phényl]-2,7-diméthoxy-3H-fluoren-3-ylidène]-N-méthyle |
| 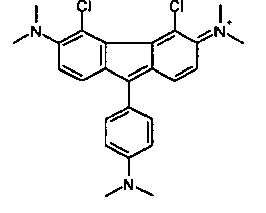 | Méthanaminium, N-[4,5-dichloro-6-(diméthylamino)-9-[4-(diméthylamino)phényl]-3H-fluoren-3-ylidène]-N-méthyle |
| 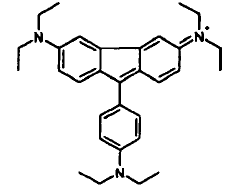 | [4-(3,6-Bis-diéthylamino-fluoren-9-ylidène)-cyclohexa-2,5-diénylidène]-diéthyl-ammonium |
| 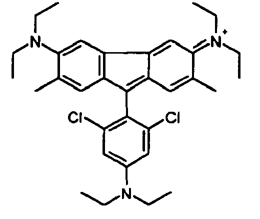 | Ethanaminium, N-[9-[2,6-dichloro-4-(diéthylamino)phényl]-6-(diéthylamino)-2,7-diméthyl-3H-fluoren-3-ylidène]-N-éthyle |
| 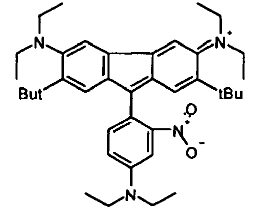 | Ethanaminium, N-[6-(diéthylamino)-9-[4-(diéthylamino)-2-nitrophényl]-2,7-bis(1,1-diméthyléthyl)-3H-fluoren-3-ylidène]-N-éthyle |
| 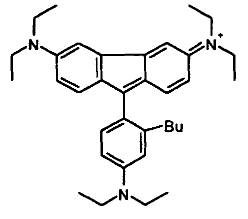 | Ethanaminium, N-[9-[2-butyl-4-(diéthylamino)phényl]-6-(diéthylamino)-3H-fluoren-3-ylidène]-N-éthyle |
| 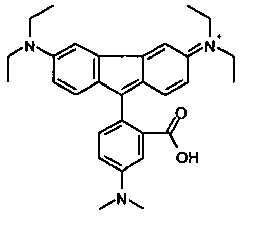 | Acide benzoïque, 2-[3,6-bis(diéthylamino)-9H-fluoren-9-yl]-5-(diméthylamino) |
| 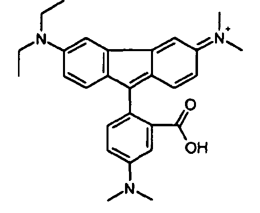 | Acide benzoïque, 2-[3-(diéthylamino)-6-(diméthylamino)-9H-fluoren-9-yl]-5-(diméthylamino) |
| 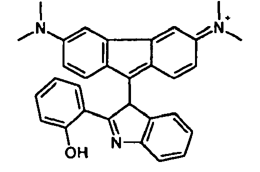 | Phénol, 2-[3-[3,6-bis(diméthylamino)-9H-fluoren-9-ylidène]-3H-indol-2-yl] |
| 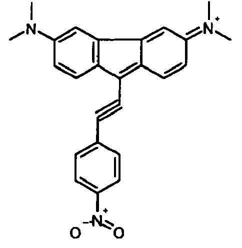 | Méthanaminium, N-[6-(diméthylamino)-9-[(4-nitrophényl)éthynyl]-3H-fluoren-3-ylidène]-N-méthyle |
| 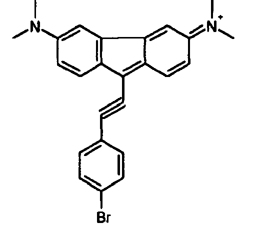 | Méthanaminium, N-[9-[(4-bromophényl)éthynyl]-6-(diméthylamino)-3H-fluoren-3-ylidène]-N-méthyle |
| 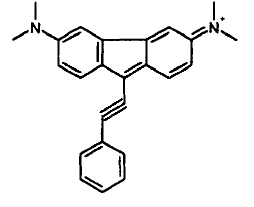 | Méthanaminium, N-[6-(diméthylamino)-9-(phénylethynyl)-3H-fluoren-3-ylidène]-N-méthyle |
| 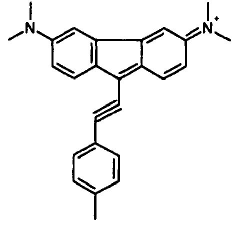 | Méthanaminium, N-[6-(diméthylamino)-9-[(4-méthylphényl)éthynyl]-3H-fluoren-3-ylidène]-N-méthyle |
| 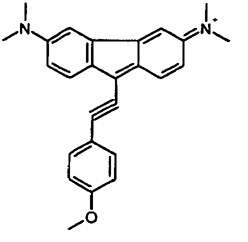 | Méthanaminium, N-[6-(diméthylamino)-9-[(4-méthoxyphényl)éthynyl]-3H-fluoren-3-ylidène]-N-méthyle |
| 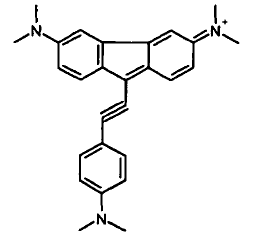 | Méthanaminium, N-[6-(diméthylamino)-9-[[4-(diméthylamino)phényl]éthynyl]-3H-fluoren-3-ylidène]-N-méthyl |

Les compositions utilisées selon la présente invention peuvent comprendre de 0,001 à 20 %, de préférence de 0,01 à 10 %, plus particulièrement 0,1 à 5% en poids de colorant(s) direct(s) dérivé(s) de triarylméthane de formule (Ia) par rapport au poids total de la composition.

L'invention porte également sur une composition tinctoriale aqueuse pour la teinture des fibres kératiniques, en particulier des fibres kératiniques humaines, telles que les cheveux comprenant dans un milieu de teinture approprié :
- au moins un colorant direct dérivé de triarylméthane tel que défini ci-dessus,
- au moins une base d'oxydation et/ou
- au moins un colorant direct différent du dérivé de triarylméthane défini ci-dessus.

La composition tinctoriale selon la présente invention peut comprendre de 0,001 à 20 %, de préférence de 0,01 à 10 % en poids de colorant(s) direct(s) dérivé(s) de triarylméthane de formule (Ia) par rapport au poids total de la composition.

La composition de la présente invention peut comprendre en outre au moins une base d'oxydation.

A titre d'exemple, les bases d'oxydation sont choisies parmi les phénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-(β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

La ou les bases d'oxydation présentes dans la composition de l'invention sont en général présentes en quantité comprise entre 0,001 et 20 % en poids environ du poids total de la composition tinctoriale, de préférence entre 0,005 et 6 % en poids.

La composition tinctoriale conforme à l'invention peut en outre contenir un ou plusieurs colorants directs additionnels autres que les colorants directs dérivés de triarylméthane de formule (Ia), pouvant notamment être choisis parmi les colorants nitrés de la série benzénique neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques utilisables selon l'invention, on peut citer de manière non limitative les composés suivants :
- 1,4-diamino-2-nitrobenzène,
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzéne
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1- β,γ-dihydroxypropyloxy-3-méthylamino-4-nitrobenzène
- 1 -β-hydroxyéthylamino-4-β,γ-dihydroxypropyloxy-2-nitrobenzène
- 1 -β,γ-dihydroxypropylamino-4-trifluorométhyl-2-nitrobenzène
- 1 -β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoïques cationiques décrits dans les demandes de brevets WO 95/15144, WO-95/01772 et EP-714954 dont le contenu fait partie intégrante de l'invention.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20 % en poids environ du poids total de la composition, et encore plus préférentiellement de 0,005 à 10 % en poids environ.

Si la composition contient au moins une base d'oxydation, la composition selon l'invention contient de préférence un ou plusieurs coupleurs conventionnellement utilisés pour la teinture de fibres kératiniques. Parmi ces coupleurs, on peut notamment citer les méta-phénylènediamines, les méta-aminphénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en quantité comprise allant de 0,001 à 20 % en poids environ du poids total de la composition tinctoriale, de préférence allant de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition selon l'invention peut également comprendre au moins un agent oxydant classiquement utilisé pour la teinture d'oxydation des fibres kératiniques, comme par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le glycérol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs et des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale conforme à l'invention est généralement compris entre 3 et 12, de préférence entre 5 et 11, et encore plus particulièrement de 6 à 8,5. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines, telles que les mono-, di- et triéthanolamines ainsi que leurs dérivés, les hydroxydes de sodium ou de potassium et les composés de formule (II) suivante : 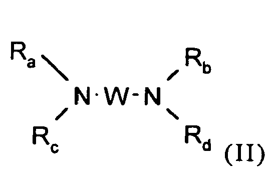 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres kératiniques la composition selon la présente invention telle que définie précédemment, on laisse pauser la composition pendant une période comprise entre 3 minutes et 1 heure, de préférence entre 15 minutes et 45 minutes, puis on rince lesdites fibres.

Selon un mode de réalisation particulier, la composition de l'invention exempte d'agent oxydant et de base d'oxydation est appliquée sur les fibres kératiniques en présence d'un agent oxydant pendant un temps suffisant pour obtenir l'éclaircissement souhaité. L'agent oxydant peut être ajouté à la composition de l'invention juste au moment de l'emploi, ou il peut être mis en oeuvre à partir d'une composition oxydante le contenant, appliquée simultanément ou séquentiellement à la composition de l'invention. Après un temps de pose de 3 minutes à 1 heure environ, de préférence entre 15 minutes et 45 minutes, les fibres kératiniques sont rincées, lavées au shampooing, rincées à nouveau puis séchées.

Selon un mode de réalisation particulier, la composition selon la présente invention exempte d'agent oxydant comprend au moins un précurseur de colorant d'oxydation, et elle est mélangée, de préférence au moment de l'emploi, à une composition contenant, dans un milieu approprié pour la coloration, au moins un agent oxydant. La composition oxydante peut être appliquée simultanément ou séquentiellement à la composition selon l'invention. Le mélange obtenu est ensuite appliqué sur les fibres kératiniques. Après un temps de pose de 3 minutes à 1 heure environ, de préférence de 15 minutes à 45 minutes environ, les fibres kératiniques sont rincées, lavées au shampooing, rincées à nouveau puis séchées.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, encore plus préférentiellement entre 5 et 11 et encore plus particulièrement entre 6 et 8,5. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention porte également sur l'utilisation de la composition tinctoriale selon l'invention pour la teinture des fibres kératiniques.

L'invention a aussi pour objet un dispositif à plusieurs compartiments ou "kit" de teinture dans lequel un premier compartiment renferme la composition tinctoriale selon l'invention exempte d'agent oxydant et de base d'oxydation et un deuxième compartiment renferme un agent oxydant. Le compartiment contenant au moins un colorant direct de formule (Ia) peut éventuellement contenir au moins un précurseur de colorant d'oxydation défini ci-dessus. Ce dispositif peut être équipé d'un moyen permettant de délivrer sur les cheveux le mélange souhaité, tel que les dispositifs décrits dans le brevet FR-2 586 913 au nom de la demanderesse.

L'invention porte également sur une composition tinctoriale aqueuse pour la teinture des fibres kératiniques, en particulier des fibres kératiniques humaines telles que les cheveux comprenant, dans un milieu de teinture approprié :
- au moins un composé leuco de formule générale (Ib) qui est un précurseur du colorant direct dérivé de triarylméthane de formule (Ia), 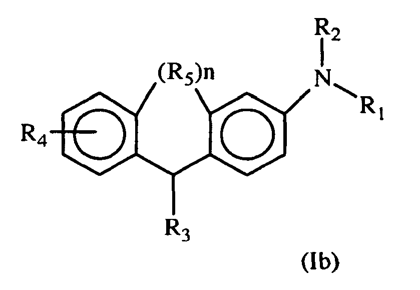
où R₁, R₂, R₃, R₄, R₅, R₆, R₇ et n ont la même signification que dans le colorant direct de formule générale (Ia),
- au moins une base d'oxydation, et/ou
- au moins un colorant direct autre que ceux de formule (Ia).

La composition tinctoriale comprenant le composé leuco de formule générale (Ib) peut également comprendre tous les constituants qui sont présents dans la composition tinctoriale comprenant le colorant direct dérivé de triarylméthane de formule générale (Ia).

L'invention porte également sur un procédé de coloration des fibres kératiniques, dans lequel on applique, sur les fibres kératiniques, la composition comprenant le composé leuco de formule (Ib) et une composition comprenant un agent oxydant afin d'oxyder le composé leuco de formule (Ib) en un colorant direct dérivé de triarylméthane de formule (Ia).

Selon un mode de réalisation particulier, la composition comprenant le composé leuco de formule (Ib) exempte d'agent oxydant et de préférence exempte de base d'oxydation est mélangée, de préférence au moment de l'emploi, à une composition contenant, dans un milieu approprié pour la coloration, au moins un agent oxydant. La composition oxydante peut être appliquée simultanément ou séquentiellement à la composition comprenant le composé leuco de formule (Ib). Le mélange obtenu est ensuite appliqué sur les fibres kératiniques. Après un temps de pose de 3 minutes à 1 heure environ, de préférence entre 15 minutes et 45 minutes, les fibres kératiniques sont rincées, lavées au shampooing, rincées à nouveau puis séchées.

L'invention porte également sur un dispositif à plusieurs compartiments ou "kit" de teinture dans lequel un premier compartiment renferme la composition tinctoriale comprenant le composé leuco de formule (Ib) exempte d'agent oxydant et de préférence exempte de base d'oxydation et un deuxième compartiment renferme un agent oxydant. Le compartiment contenant au moins un colorant direct de formule (Ia) peut éventuellement contenir au moins un précurseur de colorant d'oxydation défini ci-dessus. De la même façon que précédemment, ce dispositif peut être équipé d'un moyen permettant de délivrer sur les cheveux le mélange souhaité, tel que les dispositifs décrits dans le brevet FR-2 586 913 au nom de la demanderesse.

L'exemple suivant sert à illustrer l'invention sans toutefois présenter un caractère limitatif.

### EXEMPLE

On a préparé la composition de teinture suivante :

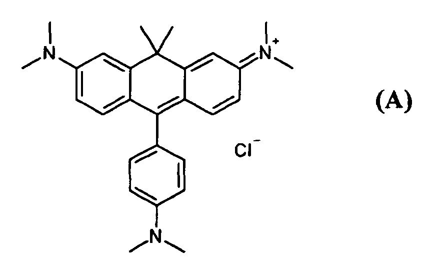

| Composé | Quantité |
|---|---|
| [7-diméthylamino-10-(4-diméthylamino-phényl)-9,9-diméthyl-9H-anthracen-2-ylidène] chlorure | 10⁻³ mol% |
| Alcool benzylique | 4,0g |
| Polyéthylèneglycol 60E | 6,0g |
| Hydroxyéthylcellulose | 0,7g |
| Alkylpolyglucoside en solution aqueuse à 60%MA* | 4,5g |
| Tampon phosphate | qsp pH7 |
| Eau déminéralisée | qsp 100g |

| | |
|---|---|
| * Matière Active | |

On a appliqué la composition ci-dessus sur des mèches de cheveux gris naturels ou permanentés à 90% de blancs avec un rapport de bain de 10 et on a laissé poser pendant 30 minutes à température ambiante. Après rinçage à l'eau courante et séchage, les cheveux ont été teints dans une nuance bleue.

## Revendications

1. Utilisation à titre de colorant direct dans une composition tinctoriale pour fibres kératiniques, en particulier les fibres kératiniques humaines telles que les cheveux, ou pour la fabrication de telles compositions, d'au moins un composé dérivé de triarylméthane de formule générale (Ia) : 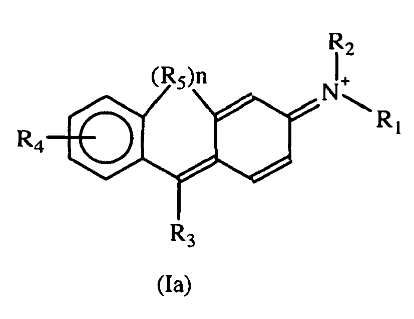 dans lesquelles :
R₁, R₂ et R₃ représentent, indépendamment les uns des autres, un atome d'hydrogène, un radical alkyle en C₁-C₂₄, alcényle ou alcynyle en C₂-C₂₄, linéaire ou ramifié, substitué ou non substitué, un radical benzyle substitué ou non substitué ou un radical aryle substitué ou non substitué, un radical hétérocycle aromatique ou non, substitué ou non,
R₄ représente un atome d'hydrogène ou un groupement amino substitué ou non substitué,
R₅ représente un groupement -CR₆R₇- dans lequel R₆ et R₇ représentent, indépendamment l'un de l'autre, un atome d'hydrogène, un radical alkyle en C₁-C₂₄, linéaire ou ramifié, substitué ou non substitué, pouvant se cycliser, un radical benzyle substitué ou non substitué ou un radical aryle substitué ou non substitué,
n désigne 0 ou 1,
le dérivé de formule (Ia) pouvant éventuellement porter au moins un autre substituant,
un radical étant dit substitué lorsqu'il porte au moins un groupement choisi parmi :
- un groupement alkyle en C₁-C₆ linéaire ou ramifié excepté lorsque les groupes R₁, R₂, R₃, R₆ et/ou R₇ portant le substituant représentent des groupements alkyle, alcényle ou alcynyle ;
- un groupement hydroxyalkyle en C₁-C₆ linéaire ou ramifié excepté lorsque les groupes R₁, R₂, R₃, R₆ et/ou R₇ portant le substituant représentent des groupements alkyle, alcényle ou alcynyle ;
- un groupement acétylamino ; un groupement alcoxy en C₁-C₆ linéaire ou ramifié ; un groupement alkylcarbonyle en C₁-C₆ linéaire ou ramifié ; un groupement hydroxycarbonyle ; un groupement alcoxycarbonyle en C₁-C₆ linéaire ou ramifié ; un groupement hydroxy ; un groupement amino ; un groupement mono ou dialkylamino ; un groupement mono ou dihydroxyalkylamino, un groupement nitro, un atome d'halogène ;
- un groupement aryle, un hétérocycle aromatique ou non, ces groupements cycliques étant non substitués ou substitués par les substituants précédemment cités.
ou ses formes mésomères.

2. Utilisation selon la revendication 1 dans laquelle le colorant direct de formule générale (Ia) est tel que R₁ et R₂ représentent, indépendamment l'un de l'autre, un atome d'hydrogène, un radical alkyle en C₁-C₆, un radical mono ou polyhydroxyalkyle en C₁-C₆.

3. Utilisation selon l'une quelconque des revendications 1 ou 2 dans laquelle le colorant direct de formule générale (Ia) est tel que R₃ représente un atome d'hydrogène ou un radical aryle éventuellement substitué par un groupement amino, dialkylamino ou dihydroxyalkyle amino.

4. Utilisation selon l'une quelconque des revendications précédentes dans laquelle le colorant direct de formule générale (Ia) est tel que n désigne 1 et R₅ désigne de préférence le groupement divalent CR₆R₇, R₆ et R₇ désignent un alkyle en C₁-C₄, éventuellement substitué par un ou plusieurs atomes d'halogène.

5. Utilisation selon l'une quelconque des revendications précédentes dans laquelle le colorant direct de formule générale (Ia) est choisi dans le groupe formé par:
- [7-Diméthylamino-9,9-diméthyl-9H-anthracen-2-ylidèn]- diméthyl ammonium;
- [7-Diméthylamino-10-éthyl-9,9-diméthyl-9H-anthracen-2-ylidèn]-diméthyl ammonium;
- [7-Diméthylamino-10-(4-diméthylamino-phényl)-9,9-diméthyl-9H-anthracen-2-ylidèn]- diméthyl ammonium;
- [7-Diéthylamino-10-(4-diéthylamino-phényl)-9,9-diméthyl-9H-anthracen-2-ylidène]-diméthyl-ammonium;
- Méthanaminium, N-[6-(diméthylamino)-9-(1-naphthalenyl)-3H-fluoren-3-ylidène]-N-méthyle;
- Méthanaminium, N-[6-(diméthylamino)-9-(4-pyridinyl)-3H-fluoren-3-ylidène]-N-méthyle;
- Méthanaminium, N-[9-[4-(diméthylamino)phenyl]-3H-fluoren-3-ylidène]-N-méthyle;
- Bis[3,6-bis(diméthylamino)-9-phénylfluoren-9-ylium];
- Acide benzoïque, 2-[3,6-bis(diméthylamino)-9H-fluoren-9-ylium];
- 3,6-Bis(diméthylamino)-9-(p-méthoxyphényl)fluoren-9-ylium;
- 4-(3,6-Diamino-fluoren-9-ylidène)-cyclohexa-2,5-diénylidène-ammonium;
- [4-(3,6-Bis-amino-fluoren-9-ylidène)-2-méthyl-cyclohexa-2,5-diénylidene]- ammonium ;
- [4-(3,6-Bis-amino-2,7-dichloro-fluoren-9-ylidène)-cyclohexa-2,5-diénylidène]- ammonium;
- 9H-Fluorène-3,6-diamine, N,N'-diméthyl-9-[4-(méthylamino) phényl];
- [4-(3,6-Bis-diméthylamino-fluoren-9-ylidène)-cyclohexa-2,5-diénylidène]-méthyl-ammonium;
- [4-(3,6-Bis-diméthylamino-fluoren-9-ylidène)-cyclohexa-2,5-diénylidène]-diméthyl-ammonium;
- [4-(3,6-Bis-diéthylamino-fluoren-9-ylidène)-cyclohexa-2,5-diénylidène]-diéthyl-ammonium;
- Ethanaminium, N-[9-[2,6-dichloro-4-(diéthylamino)phényl]-6-(diéthylamino)-2,7-diméthyl-3H-fluoren-3-ylidène]-N-éthyle;
- Ethanaminium, N-[6-(diéthylamino)-9-[4-(diéthylamino)-2-nitrophényl]-2,7-bis(1,1-diméthyléthyl)-3H-fluoren-3-ylidène]-N-éthyle ;
- Ethanaminium, N-[9-[2-butyl-4-(diéthylamino)phényl]-6-(diéthylamino)-3H-fluoren-3-ylidène]-N-éthyle ;
- Acide benzoïque, 2-[3,6-bis(diéthylamino)-9H-fluoren-9-yl]-5-(diméthylamino);
- Acide benzoïque, 2-[3-(diéthylamino)-6-(diméthylamino)-9H-fluoren-9-yl]-5-(diméthylamino);
- Phénol, 2-[3-[3,6-bis(diméthylamino)-9H-fluoren-9-ylidène]-3H-indol-2-yl];
- Méthanaminium, N-[6-(diméthylamino)-9-[(4-nitrophényl)éthynyl]-3H-fluoren-3-ylidène]-N-méthyle;
- Méthanaminium, N-[9-[(4-bromophényl)éthynyl]-6-(diméthylamino)-3H-fluoren-3-ylidène]-N-méthyle;
- Méthanaminium, N-[6-(diméthylamino)-9-(phénylethynyl)-3H-fluoren-3-ylidène]-N-méthyle;
- Méthanaminium, N-[6-(diméthylamino)-9-[(4-méthylphényl)éthynyl]-3H-fluoren-3-ylidène]-N-méthyle;
- Méthanaminium, N-[6-(diméthylamino)-9-[(4-méthoxyphényl)éthynyl]-3H-fluoren-3-ylidène]-N-méthyle;
- Méthanaminium, N-[6-(diméthylamino)-9-[[4-(diméthylamino)phényl]éthynyl]-3H-fluoren-3-ylidène]-N-méthyl; et
- 9H-Fluorène-3,6-diamine, 9-(4-pyridinylméthylène).

6. Utilisation selon l'une quelconque des revendications précédentes dans laquelle le colorant direct dérivé de triarylméthane de formule (Ia) est présent dans la composition tinctoriale en une quantité allant de 0,001 à 20 %, de préférence de 0,01 à 10% et de manière encore préférée de 0,1 à 5 % de colorant(s) direct(s) de formule (Ia) par rapport au poids total de la composition.

7. Composition tinctoriale aqueuse pour la teinture des fibres kératiniques, en particulier des fibres kératiniques humaines telles que les cheveux comprenant, dans un milieu de teinture approprié :
- au moins un colorant direct dérivé de triarylméthane de formule générale (Ia) tel que défini à l'une quelconque des revendications 1 à 6,
- au moins une base d'oxydation, et/ou
- au moins un colorant direct autre que ceux de formule (Ia).

8. Composition tinctoriale selon la revendication 7, telle qu'elle comprend de 0,001 à 20 %, de préférence de 0,01 à 10 % de colorant(s) direct(s) de formule (Ia) par rapport au poids total de la composition.

9. Composition tinctoriale selon la revendications 7 ou 8, dans laquelle la base d'oxydation est choisie parmi les para-phénylènediamines, les bis-phénylalkylènediamines, les para-aminophénols, les ortho-aminophénols, les bases hétérocycliques et leurs sels d'addition.

10. Composition tinctoriale selon la revendication 9, dans laquelle la ou les bases d'oxydation sont présentes en une quantité comprise entre 0,001 à 20 % en poids et de préférence entre 0,005 et 6% en poids par rapport au poids total de la composition.

11. Composition tinctoriale selon l'une quelconque des revendications 7 à 10 telle qu'elle comprend en outre un ou plusieurs colorants directs additionnels autres que les composés de formule (Ia), choisis parmi les colorants nitrés de la série benzénique neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

12. Composition tinctoriale selon la revendication 11, dans laquelle la ou les colorants directs additionnels sont présents en une quantité comprise entre 0,001 à 20 % en poids et de préférence entre 0,005 et 10% en poids par rapport au poids total de la composition.

13. Composition tinctoriale selon l'une quelconque des revendications 7 à 12, telle qu'elle comprend en outre un coupleur choisi parmi les méta-phénylènediamines, les méta-aminophénols, les méta-diphénols, les coupleurs naphtaléniques, les coupleurs hétérocycliques et leurs sels d'addition.

14. Composition selon la revendication 13, telle que le coupleur est choisi parmi le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

15. Composition selon la revendication 13 ou 14, telle que le ou les coupleurs sont présents en une quantité comprise entre 0,001 et 20 %, de préférence entre 0,005 et 6 % en poids par rapport au poids total de la composition.

16. Composition selon l'une des revendications 7 à 15, telle qu'elle comprend au moins un agent oxydant choisi parmi le peroxyde d'hydrogène, le peroxyde d'urée, les bromates de métaux alcalins, les persels, les peracides et les enzymes oxydases, et de préférence le peroxyde d'hydrogène.

17. Composition selon l'une quelconque des revendications 7 à 16, telle qu'elle comprend au moins un solvant hydroxylé tel que l'éthanol, le propylène glycol, le glycérol, les mono éthers de polyols.

18. Composition selon l'une quelconque des revendications 7 à 17, telle qu'elle comprend au moins un adjuvant choisi parmi les agents tensio-actifs anioniques, cationiques, non-ioniques, amphotères, zwitterioniques ou leurs mélanges, les polymères anioniques, cationiques, non-ioniques, amphotères, zwitterioniques ou leurs mélanges, les agents épaississants minéraux ou organiques, et en particulier les épaississants associatifs anioniques, cationiques, non ioniques et amphotères, les agents antioxydants, les agents de pénétration, les agents séquestrants, les parfums, les tampons, les agents dispersants, les agents conditionneurs tels que les silicones volatiles ou non volatiles, modifiées ou non modifiées, les agents filmogènes, les céramides, les agents conservateurs, les agents opacifiants.

19. Procédé de coloration des fibres kératiniques, dans lequel on applique la composition cosmétique telle que définie dans l'une quelconque des revendications 7 à 18 sur les fibres kératiniques, on laisse pauser la composition pendant une période comprise entre 3 minutes et 1 heure et de préférence entre 15 minutes et 45 minutes, puis on rince lesdites fibres.

20. Procédé d'éclaircissement des fibres kératiniques, dans lequel on applique la composition cosmétique telle que définie dans l'une quelconque des revendications 7 à 18 exempte d'agent oxydant et de base d'oxydation sur les fibres kératiniques, on applique simultanément ou séquentiellement à la composition de coloration une composition oxydante, on laisse pauser les compositions pendant une période comprise entre 3 minutes et 1 heure et de préférence entre 15 minutes et 45 minutes, puis on rince, lave au shampooing, rince à nouveau et sèche lesdites fibres.

21. Procédé de coloration des fibres kératiniques, dans lequel on applique la composition cosmétique telle que définie dans l'une quelconque des revendications 7 à 18 comprenant au moins un précurseur de colorant d'oxydation et exempte d'agent oxydant sur les fibres kératiniques, on applique simultanément ou séquentiellement à la composition de coloration une composition oxydante, on laisse pauser les compositions pendant une période comprise entre 3 minutes et 1 heure et de préférence entre 15 minutes et 45 minutes, puis on rince, lave au shampooing, rince à nouveau et sèche lesdites fibres.

22. Dispositif à plusieurs compartiments ou « Kit » de coloration, **caractérisé par le fait qu'**il comporte un premier compartiment renfermant une composition telle que définie selon l'une des revendications 7 à 18 exempte d'agent oxydant et de base d'oxydation et un second compartiment renfermant une composition comprenant un agent oxydant.

23. Dispositif selon la revendication 22, **caractérisé par le fait que** le premier compartiment comporte un précurseur de colorant d'oxydation.

24. Utilisation d'une composition selon l'une des revendications 7 à 18 pour la teinture des fibres kératiniques.

25. Composition tinctoriale aqueuse pour la teinture des fibres kératiniques, en particulier des fibres kératiniques humaines telles que les cheveux comprenant, dans un milieu de teinture approprié :
- au moins un composé leuco de formule (Ib), précurseur du composé de formule (Ia) tel que défini à l'une quelconque des revendications 1 à 6, 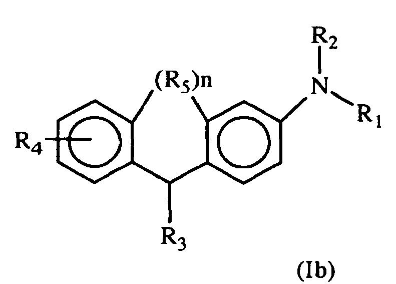
où R₁, R₂, R₃, R₄, R₅, R₆, R₇ et n ont la même signification que dans le colorant direct dérivé de triarylméthane de formule (Ia),
- au moins une base d'oxydation, et/ou
- au moins un colorant direct autre que ceux de formule (Ia).

26. Procédé de coloration des fibres kératiniques, dans lequel on applique la composition cosmétique telle que définie dans la revendication 25, exempte d'agent oxydant sur les fibres kératiniques, on applique simultanément ou séquentiellement à la composition cosmétique une composition oxydante, on laisse pauser les compositions pendant une période comprise entre 3 minutes et 1 heure et, de préférence entre 15 minutes et 45 minutes, puis on rince, lave au shampooing, rince à nouveau et sèche lesdites fibres.

27. Dispositif à plusieurs compartiments ou « Kit » de coloration, **caractérisé par le fait qu'**il comporte un premier compartiment renfermant une composition telle que définie selon la revendication 25, exempte d'agent oxydant et un second compartiment renfermant une composition comprenant un agent oxydant.

## Claims

1. Use as direct dye in a dye composition for keratin fibres, in particular human keratin fibres such as the hair, or for the manufacture of such compositions, of at least one triarylmethane-based compound of general formula (Ia): 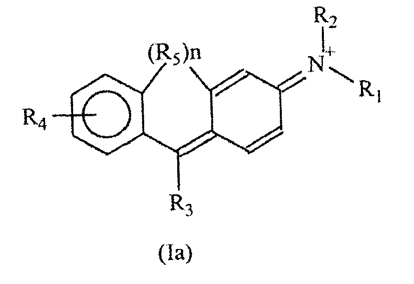 in which:
R₁, R₂ and R₃ represent, independently of each other, a hydrogen atom, a linear or branched, substituted or unsubstituted C₁-C₂₄ alkyl or C₂-C₂₄ alkenyl or alkynyl radical, a substituted or unsubstituted benzyl radical, a substituted or unsubstituted aryl radical or an aromatic or nonaromatic, substituted or unsubstituted heterocycle radical,
R₄ represents a hydrogen atom or a substituted or unsubstituted amino group,
R₅ represents a group -CR₆R₇- in which R₆ and R₇ represent, independently of each other, a hydrogen atom, a linear or branched, substituted or unsubstituted C₁-C₂₄ alkyl radical, which can cyclize, a substituted or unsubstituted benzyl radical or a substituted or unsubstituted aryl radical, n denotes 0 or 1,
the derivative of formula (Ia) possibly bearing at least one other substituent,
a radical being said to be substituted when it bears at least one group chosen from :
- a linear or branched C₁-C₆ alkyl group, except when the groups R₁, R₂, R₃, R₆ and/or R₇ bearing the substituent represent alkyl, alkenyl or alkynyl groups;
- a linear or branched C₁-C₆ hydroxyalkyl group, except when the groups R₁, R₂, R₃, R₆ and R₇ bearing the substituent represent alkyl, alkenyl or alkynyl groups;
- an acetylamino group; a linear or branched C₁-C₆ alkoxy group; a linear or branched C₁-C₆ alkylcarbonyl group; a hydroxycarbonyl group; a linear or branched C₁-C₆ alkoxycarbonyl group; a hydroxyl group; an amino group; a mono- or dialkylamino group; a mono- or dihydroxyalkylamino group, a nitro group, a halogen atom;
- an aryl group, an aromatic or non-aromatic heterocycle, these cyclic groups being unsubstituted or substituted with the substituents mentioned above, or mesomeric forms thereof.

2. Use according to Claim 1, in which the direct dye of general formula (Ia) is such that R₁ and R₂ represent, independently of each other, a hydrogen atom, a C₁-C₆ alkyl radical or a C₁-C₆ mono- or polyhydroxyalkyl radical.

3. Use according to either of Claims 1 and 2, in which the direct dye of general formula (Ia) is such that R₃ represents a hydrogen atom or an aryl radical, optionally substituted with an amino, dialkylamino or dihydroxyalkylamino group.

4. Use according to any one of the preceding claims, in which the direct dye of general formula (Ia) is such that n denotes 1 and R₅ preferably denotes the divalent group CR₆R₇, R₆ and R₇ denoting a C₁-C₄ alkyl, optionally substituted with one or more halogen atoms.

5. Use according to any one of the preceding claims, in which the direct dye of general formula (Ia) is chosen from the group formed by:
- [7-Dimethylamino-9,9-dimethyl-9H-anthracen-2-ylidene]dimethylammonium;
- [7-Dimethylamino-10-ethyl-9,9-dimethyl-9H-anthracen-2-ylidene]dimethylammonium;
- [7-Dimethylamino-10-(4-dimethylaminophenyl)-9,9-dimethyl-9H-anthracen-2-ylidene]dimethylammonium;
- [7-Diethylamino-10-(4-diethylaminophenyl)-9,9-dimethyl-9H-anthracen-2-ylidene]dimethylammonium;
- Methanaminium, N-[6-(dimethylamino)-9-(1-naphthalenyl)-3H-fluoren-3-ylidene]-N-methyl;
- Methanaminium, N-[6-(dimethylamino)-9-(4-pyridyl)-3H-fluoren-3-ylidene]-N-methyl;
- Methanaminium, N-[9-[4-(dimethylamino)phenyl]-3H-fluoren-3-ylidene]-N-methyl;
- Bis[3,6-bis(dimethylamino)-9-phenylfluoren-9-ylium];
- Benzoic acid, 2-[3,6-bis(dimethylamino)-9H-fluoren-9-ylium];
- 3,6-Bis(dimethylamino)-9-(p-methoxyphenyl)fluoren-9-ylium;
- 4-(3,6-Diaminofluoren-9-ylidene)cyclohexa-2,5-dienylideneammonium;
- [4-(3,6-Bis-aminofluoren-9-ylidene)-2-methylcyclohexa-2,5-dienylidene]ammonium;
- [4-(3,6-Bis-amino-2,7-dichlorofluoren-9-ylidene)cyclohexa-2,5-dienylidene]ammonium;
- 9H-Fluorene-3,6-diamine, N,N'-dimethyl-9-[4-(methylamino)phenyl];
- [4-(3,6-Bis-dimethylaminofluoren-9-ylidene)cyclohexa-2,5-dienylidene]methylammonium;
- [4-(3,6-Bis-dimethylaminofluoren-9-ylidene)cyclohexa-2,5-dienylidene]dimethylammonium;
- [4-(3,6-Bis-diethylaminofluoren-9-ylidene)cyclohexa-2,5-dienylidene]diethylammonium;
- Ethanaminium, N-[9-[2,6-dichloro-4-(diethylamino)phenyl]-6-(diethylamino)-2,7-dimethyl-3H-fluoren-3-ylidene]-N-ethyl;
- Ethanaminium, N-[6-(diethylamino)-9-[4-(diethylamino)-2-nitrophenyl]-2,7-bis(1,1-dimethylethyl)-3H-fluoren-3-ylidene]-N-ethyl;
- Ethanaminium, N-[9-[2-butyl-4-(diethylamino)phenyl]-6-(diethylamino)-3H-fluoren-3-ylidene]-N-ethyl;
- Benzoic acid, 2-[3,6-bis(diethylamino)-9H-fluoren-9-yl]-5-(dimethylamino);
- Benzoic acid, 2-[3-(diethylamino)-6-(dimethylamino)-9H-fluoren-9-yl]-5-(dimethylamino);
- Phenol, 2-[3-[3,6-bis(dimethylamino)-9H-fluoren-9-ylidene]-3H-indol-2-yl];
- Methanaminium, N-[6-(dimethylamino)-9-[(4-nitrophenyl)ethynyl]-3H-fluoren-3-ylidene]-N-methyl;
- Methanaminium, N-[9-[(4-bromophenyl)ethynyl]-6-(dimethylamino)-3H-fluoren-3-ylidene]-N-methyl;
- Methanaminium, N-[6-(dimethylamino)-9-(phenylethynyl)-3H-fluoren-3-ylidene]-N-methyl;
- Methanaminium, N-[6-(dimethylamino)-9-[(4-methylphenyl)ethynyl]-3H-fluoren-3-ylidene]-N-methyl;
- Methanaminium, N-[6-(dimethylamino)-9-[(4-methoxyphenyl)ethynyl]-3H-fluoren-3-ylidene]-N-methyl;
- Methanaminium, N-[6-(dimethylamino)-9-[[4-(dimethylamino)phenyl]ethynyl]-3H-fluoren-3-ylidene]-N-methyl; and
- 9H-Fluorene-3,6-diamine, 9-(4-pyridylmethylene).

6. Use according to any one of the preceding claims, in which the triarylmethane-based direct dye of formula (Ia) is present in the dye composition in an amount ranging from 0.001% to 20%, preferably from 0.01% to 10% and even more preferably from 0.1% to 5% of direct dye(s) of formula (Ia) relative to the total weight of the composition.

7. Aqueous dye composition for dyeing keratin fibres, in particular human keratin fibres such as the hair, comprising, in a suitable dyeing medium:
- at least one triarylmethane-based direct dye of general formula (Ia) as defined in any one of Claims 1 to 6,
- at least one oxidation base, and/or
- and/or at least one direct dye other than those of formula (Ia).

8. Dye composition according to Claim 7, such that it comprises from 0.001% to 20% and preferably from 0.01% to 10% of direct dye(s) of formula (Ia) relative to the total weight of the composition.

9. Dye composition according to Claim 7, in which the oxidation base is chosen from para-phenylenediamines, bis(phenyl)alkylenediamines, para-aminophenols, ortho-aminophenols and heterocyclic bases, and the addition salts thereof.

10. Dye composition according to Claim 9, in which the oxidation base(s) is (are) present in an amount of between 0.001% and 20% by weight and preferably between 0.005% and 6% by weight relative to the total weight of the composition.

11. Dye composition according to any one of Claims 7 to 10, such that it also comprises one or more additional direct dyes other than the compounds of formula (Ia), chosen from neutral, acidic or cationic nitrobenzene dyes, neutral, acidic or cationic azo direct dyes, neutral, acidic or cationic quinone and in particular anthraquinone direct dyes, azine direct dyes, triarylmethane direct dyes, indoamine direct dyes and natural direct dyes.

12. Dye composition according to Claim 11, in which the additional direct dye(s) is(are) present in an amount of between 0.001% and 20% by weight and preferably between 0.005% and 10% by weight relative to the total weight of the composition.

13. Dye composition according to any one of Claims 7 to 12, such that it also comprises a coupler chosen from meta-phenylenediamines, meta-aminophenols, meta-diphenols, naphthalene-based couplers and heterocyclic couplers, and the addition salts thereof.

14. Composition according to Claim 13, such that the coupler is chosen from 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminoenzene, 1,3-bis(2,4-diaminophenoxy)propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-β-hydroxyethyl)amino-3,4- methylenedioxybenzene and 2,6-bis (β-hydroxyethylamino) toluene, and the addition salts thereof.

15. Composition according to Claim 13 or 14, such that the coupler(s) is (are) present in an amount of between 0.001% and 20% and preferably between 0.005% and 6% by weight relative to the total weight of the composition.

16. Composition according to one of Claims 7 to 15, such that it comprises at least one oxidizing agent chosen from hydrogen peroxide, urea peroxide, alkali metal bromates, persalts, peracids and oxidase enzymes, and preferably hydrogen peroxide.

17. Composition according to any one of Claims 7 to 16, such that it comprises at least one hydroxylated solvent such as ethanol, propylene glycol, glycerol or polyol monoethers.

18. Composition according to any one of Claims 7 to 17, such that it comprises at least one adjuvant chosen from anionic, cationic, nonionic, amphoteric or zwitterionic surfactants or mixtures thereof, anionic, cationic, nonionic, amphoteric or zwitterionic polymers or mixtures thereof, mineral or organic thickeners, and in particular anionic, cationic, nonionic and amphoteric associative thickeners, antioxidants, penetrating agents, sequestering agents, fragrances, buffers, dispersants, conditioners, such as volatile or non-volatile, modified or unmodified silicones, film-forming agents, ceramides, preserving agents and opacifiers.

19. Process for dyeing keratin fibres, in which the cosmetic composition as defined in any one of Claims 7 to 18 is applied to the keratin fibres, the composition is left on the fibres for a period of between 3 minutes and 1 hour and preferably between 15 minutes and 45 minutes, and the said fibres are rinsed.

20. Process for lightening keratin fibres, in which the cosmetic composition as defined in any one of Claims 7 to 18, free of oxidizing agent and of oxidation base, is applied to the keratin fibres, an oxidizing composition is applied simultaneously with or sequentially to the dye composition, the compositions are left on the fibres for a period of between 3 minutes and 1 hour and preferably between 15 minutes and 45 minutes, and the said fibres are then rinsed, washed with shampoo, rinsed again and dried.

21. Process for dyeing keratin fibres, in which the cosmetic composition as defined in any one of Claims 7 to 18, comprising at least one oxidation dye precursor and free of oxidizing agent, is applied to the keratin fibres, an oxidizing composition is applied simultaneously with or sequentially to the dye composition, the compositions are left on the fibres for a period of between 3 minutes and 1 hour and preferably between 15 minutes and 45 minutes, and the said fibres are then rinsed, washed with shampoo, rinsed again and dried.

22. Multi-compartment device or dyeing "kit", **characterized in that** it comprises a first compartment containing a composition as defined according to one of Claims 7 to 18, free of oxidizing agent and of oxidation base, and a second compartment containing a composition comprising an oxidizing agent.

23. Device according to Claim 22, **characterized in that** the first compartment comprises an oxidation dye precursor.

24. Use of a composition according to one of Claims 7 to 18 for dyeing keratin fibres.

25. Aqueous dye composition for dyeing keratin fibres, in particular human keratin fibres such as the hair, comprising, in a suitable dyeing medium:
- at least one leuco compound of formula (Ib), which is a precursor of the compound of formula (Ia) as defined in any one of Claims 1 to 6, 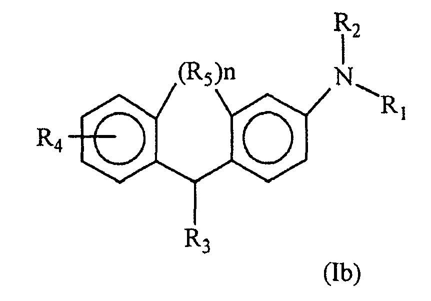
in which R₁, R₂, R₃, R₄, R₅, R₆, R₇ and n have the same meaning as in the triarylmethane-based direct dye of formula (Ia),
- at least one oxidation base, and/or
- at least one direct dye other than those of formula (Ia).

26. Process for dyeing keratin fibres, in which the cosmetic composition as defined in Claim 25, free of oxidizing agent, is applied to the keratin fibres, an oxidizing composition is applied simultaneously with or sequentially to the cosmetic composition, the compositions are left on the fibres for a period of between 3 minutes and 1 hour and preferably between 15 minutes and 45 minutes, and the said fibres are then rinsed, washed with shampoo, rinsed again and dried.

27. Multi-compartment device or dyeing "kit", **characterized in that** it comprises a first compartment containing a composition as defined according to Claim 25, free of oxidizing agent, and a second compartment containing a composition comprising an oxidizing agent.

## Patentansprüche

1. Verwendung als Direktfarbstoff in einer Färbezusammensetzung für Keratinfasern, insbesondere für menschliche Keratinfasern, wie Haare, oder zur Herstellung dieser Zusammensetzungen mindestens einer von Triarylmethan stammenden Verbindung der allgemeinen Formel (Ia): 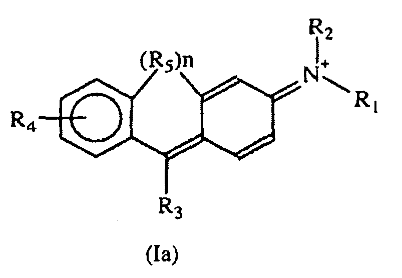 worin:
R₁, R₂ und R₃ unabhängig voneinander ein Wasserstoffatom, einen geraden oder verzweigten, substituierten oder nicht substituierten C₁-C₂₄-Alkylrest, C₂-C₂₄-Alkenyl- oder -Alkinylrest, einen substituierten oder nicht substituierten Benzylrest oder einen substituierten oder nicht substituierten Arylrest, einen aromatischen oder nicht aromatischen, substituierten oder nicht substituierten Heterocyclylrest darstellen,
R₄ ein Wasserstoffatom oder eine substituierte oder nicht substituierte Aminogruppe darstellt,
R₅ eine Gruppe -CR₆R₇- darstellt, worin R₆ und R₇ unabhängig voneinander ein Wasserstoffatom, einen geraden oder verzweigten, substituierten oder nicht substituierten C₁-C₂₄-Alkylrest, der cyclisieren kann, einen substituierten oder nicht substituierten Benzylrest oder einen substituierten oder nicht substituierten Arylrest darstellen,
n für 0 oder 1 steht,
wobei das Derivat der Formel (Ia) gegebenenfalls mindestens einen weiteren Substituenten tragen kann,
wobei ein Rest als substituiert bezeichnet wird, wenn er mindestens eine Gruppe trägt, die ausgewählt ist aus:
- einer geraden oder verzweigten C₁-C₆-Alkylgruppe, ausgenommen wenn die Gruppen R₁, R₂, R₃, R₆ und/oder R₇ den Substituenten tragen, der die Alkyl-, Alkenyl- oder Alkinylgruppen darstellt;
- einer geraden oder verzweigten C₁-C₆-Hydroxyalkylgruppe, ausgenommen wenn die Gruppen R₁, R₂, R₃, R₆ und/oder R₇ den Substituenten tragen, der die Alkyl-, Alkenyl- oder Alkinylgruppen darstellt;
- einer Acetylaminogruppe; einer geraden oder verzweigten C₁-C₆-Alkoxygruppe; einer geraden oder verzweigten C₁-C₆-Alkylcarbonylgruppe, einer Hydroxycarbonylgruppe; einer geraden oder verzweigten C₁-C₆-Alkoxycarbonylgruppe; einer Hydroxygruppe; einer Aminogruppe; einer Mono- oder Dialkylaminogruppe; einer Mono- oder Dihydroxyalkylaminogruppe; einer Nitrogruppe, einem Halogenatom;
- einer Arylgruppe, einem aromatischen oder nicht aromatischen Heterocyclus, wobei diese cyclischen Gruppen mit den vorstehend genannten Substituenten substituiert oder nicht substituiert sind,
oder ihren mesomeren Formen.

2. Verwendung nach Anspruch 1, wobei der Direktfarbstoff der allgemeinen Formel (Ia) derjenige ist, worin R₁ und R₂ unabhängig voneinander ein Wasserstoffatom, einen C₁-C₆-Alkylrest, einen Mono- oder Polyhydroxy-C₁-C₆-alkylrest darstellen.

3. Verwendung nach einem der Ansprüche 1 oder 2, wobei der Direktfarbstoff der allgemeinen Formel (Ia) derjenige ist, worin R₃ ein Wasserstoffatom oder einen Arylrest darstellt, der gegebenenfalls mit einer Amino-, Dialkylamino- oder Dihydroxyalkylaminogruppe substituiert ist.

4. Verwendung nach einem der vorstehenden Ansprüche, wobei der Direktfarbstoff der allgemeinen Formel (Ia) derjenige ist, worin n für 1 steht und R₅ vorzugsweise die zweiwertige Gruppe CR₆R₇ darstellt, worin R₆ und R₇ einen C₁-C₄-Alkylrest bedeuten, der gegebenenfalls mit einem oder mehreren Halogenatomen substituiert ist.

5. Verwendung nach einem der vorstehenden Ansprüche, wobei der Direktfarbstoff der allgemeinen Formel (Ia) aus der durch die Folgenden gebildeten Gruppe ausgewählt ist:
- [7-Dimethylamino-9,9-dimethyl-9H-anthracen-2-yliden]-dimethylammonium;
- [7-Dimethylamino-10-ethyl-9,9-dimethyl-9H-anthracen-2-yliden]-dimethylammonium;
- [7-Dimethylamino-10-(4-dimethylaminophenyl)-9,9-dimethyl-9H-anthracen-2-yliden]-dimethylammonium;
- [7-Diethylamino-10-(4-diethylaminophenyl)-9,9-dimethyl-9H-anthracen-2-yliden]-dimethylammonium;
- Methanaminium, N- [6-(Dimethylamino)-9-(1-naphthalinyl)-3H-fluoren-3-yliden]-N-methyl;
- Methanaminium, N-[6-(Dimethylamino)-9-(4-pyridinyl)-3H-fluoren-3-yliden]-N-methyl;
- Methanaminium, N-[9-[4-(Dimethylamino)-phenyl]-3H-fluoren-3-yliden]-N-methyl;
- Bis-[3,6-bis-(dimethylamino)-9-phenylfluoren-9-ylium];
- Benzoesäure, 2-[3,6-Bis(dimethylamino)-9H-fluoren-9-ylium];
- 3,6-Bis(dimethylamino)-9-(p-methoxyphenyl)-fluoren-9-ylium;
- 4-(3,6-Diaminofluoren-9-yliden)-cyclohexa-2,5-dienylidenammonium;
- [4-(3,6-Bisaminofluoren-9-yliden)-2-methyl-cyclohexa-2,5-dienyliden]-ammonium;
- [4-(3,6-Bisamino-2,7-dichlorfluoren-9-yliden)-cyclohexa-2,5-dienyliden]-ammonium;
- 9H-Fluoren-3,6-diamin, N,N'-Dimethyl-9-[4-(methylamino)-phenyl];
- [4-(3,6-Bisdimethylaminofluoren-9-yliden)-cyclohexa-2,5-dienyliden]-methylammonium;
- [4-(3,6-Bisdimethylaminofluoren-9-yliden)-cyclohexa-2,5-dienyliden]-dimethylammonium;
- [4-(3,6-Bisdiethylaminofluoren-9-yliden)-cyclohexa-2,5-dienyliden]-diethylammonium;
- Ethanaminium, N-[9-[2,6-Dichlor-4-(diethyl-amino)-phenyl]-6-(diethylamino)-2,7-dimethyl-3H-fluoren-3-yliden]-N-ethyl;
- Ethanaminium, N-[6-(Diethylamino)-9-[4-(diethylamino)-2-nitrophenyl]-2,7-bis-(1,1-dimethylethyl)-3H-fluoren-3-yliden]-N-ethyl;
- Ethanaminium, N-[9-[2-Butyl-4-(diethylamino)-phenyl]-6-(diethylamino)-3H-fluoren-3-yliden]-N-ethyl;
- Benzoesäure, 2-[3, 6-Bis-(diethylamino)-9H-fluoren-9-yl]-5-(dimethylamino);
- Benzoesäure, 2-[3-(Diethylamino)-6-(dimethylamino)-9H-fluoren-9-yl]-5-(dimethylamino);
- Phenol, 2-[3-[3,6-Bis(dimethylamino)-9H-fluoren-9-yliden]-3H-indol-2-yl];
- Methanaminium, N-[6-(Dimethylamino)-9-[(4-nitrophenyl)-ethinyl]-3H-fluoren-3-yliden]-N-methyl;
- Methanaminium, N-[9-[(4-Bromphenyl)-ethinyl]-6-(dimethylamino)-3H-fluoren-3-yliden]-N-methyl;
- Methanaminium, N-[6-(Dimethylamino)-9-(phenylethinyl)-3H-fluoren-3-yliden]-N-methyl;
- Methanaminium, N-[6-(Dimethylamino)-9-[(4-methylphenyl)-ethinyl]-3H-fluoren-3-yliden]-N-methyl;
- Methanaminium, N-[6-(Dimethylamino)-9-[(4-methoxyphenyl)-ethinyl]-3H-fluoren-3-yliden]-N-methyl;
- Methanaminium, N-[6-(Dimethylamino)-9-[[4-(dimethylamino)-phenyl]-ethinyl]-3H-fluoren-3-yliden]-N-methyl und
- 9H-Fluoren-3,6-diamin, 9-(4-Pyridinylmethylen).

6. Verwendung nach einem der vorstehenden Ansprüche, wobei der Triarylmethanderivat-Direktfarbstoff der allgemeinen Formel (Ia) in der Färbezusammensetzung in einer Menge von 0,001 bis 20%, vorzugsweise von 0,01 bis 10% und noch stärker bevorzugt von 0,1 bis 5% Direktfarbstoff(e) der Formel (Ia), bezogen auf das Gesamtgewicht der Zusammensetzung, vorliegt.

7. Wässrige Färbezusammensetzung zum Färben von Keratinfasern, insbesondere menschlichen Keratinfasern, wie Haaren, umfassend in einem geeigneten Färbemedium:
- mindestens einen Triarylmethanderivat-Direktfarbstoff der allgemeinen Formel (Ia) nach einem der Ansprüche 1 bis 6,
- mindestens eine Oxidationsbase und/oder
- mindestens ein anderes Färbemittel als diejenigen der Formel (Ia).

8. Färbezusammensetzung nach Anspruch 7, die 0,001 bis 20%, vorzugsweise 0,01 bis 10%, Direktfarbstoff(e) der Formel (Ia), bezogen auf das Gesamtgewicht der Zusammensetzung, umfasst.

9. Färbezusammensetzung nach den Ansprüchen 7 oder 8, wobei die Oxidationsbase aus para-Phenylendiaminen, Bisphenylalkylendiaminen, para-Aminophenolen, ortho-Aminophenolen, heterocyclischen Basen und ihren Additionssalzen ausgewählt ist.

10. Färbezusammensetzung nach Anspruch 9, worin die Oxidationsbase(n) in einer Menge zwischen 0,001 und 20 Gew.-% und vorzugsweise zwischen 0,005 und 6 Gew.-%, bezogen auf das Gesamtgewicht der Zusammensetzung, vorliegen.

11. Färbezusammensetzung nach einem der Ansprüche 7 bis 10, die außerdem einen oder mehrere zusätzliche andere Direktfarbstoffe als die Verbindungen der Formel (Ia) umfasst, die aus den nitrierten Farbstoffen der neutralen, sauren oder kationischen Benzol-Reihe, den neutralen, sauren oder kationischen Azo-Direktfarbstoffen, den neutralen, sauren oder kationischen Chinon- und insbesondere Anthrachinon-Direktfarbstoffen, den Azin-Direktfarbstoffen, den Triarylmethan-Direktfarbstoffen, den Indoamin-Direktfarbstoffen und den natürlichen Direktfarbstoffen ausgewählt sind.

12. Färbezusammensetzung nach Anspruch 11, worin der (die) zusätzliche(n) Direktfarbstoff(e) in einer Menge zwischen 0,001 und 20 Gew.-% und vorzugsweise zwischen 0,005 und 10 Gew.-%, bezogen auf das Gesamtgewicht der Zusammensetzung, vorliegen.

13. Färbezusammensetzung nach einem der Ansprüche 7 bis 12, die außerdem einen Kuppler umfasst, der aus meta-Phenylendiaminen, meta-Aminophenolen, meta-Diphenolen, naphthalinischen Kupplern, heterocyclischen Kupplern und ihren Additionssalzen ausgewählt ist.

14. Zusammensetzung nach Anspruch 13, wobei der Kuppler aus 1,3-Dihydroxybenzol, 1,3-Dihydroxy-2-methylbenzol, 4-Chlor-1,3-dihydroxybenzol, 2,4-Diamino-1-(β-hydroxyethyloxy)-benzol, 2-Amino-4-(β-hydroxy-ethylamino)-1-methoxybenzol, 1,3-Diaminobenzol, 1,3-Bis-(2,4-diaminophenoxy)-propan, 3-Ureidoanilin, 3-Ureido-1-dimethylaminobenzol, Sesamol, 1-β-Hydroxyethylamino-3,4-methylendioxybenzol, α-Naphthol, 2-Methyl-1-naphthol, 6-Hydroxyindol, 4-Hydroxyindol, 4-Hydroxy-N-methylindol, 2-Amino-3-hydroxypyridin, 6-Hydroxybenzomorpholin, 3,5-Diamino-2,6-dimethoxypyridin, 1-N-(β-Hydroxyethyl)-amino-3,4-methylendioxybenzol, 2,6-Bis-(β-hydroxyethylamino)-toluol und ihren Additionssalzen ausgewählt ist.

15. Zusammensetzung nach Anspruch 13 oder 14, worin der oder die Kuppler in einer Menge zwischen 0,001 und 20 Gew.-%, vorzugsweise zwischen 0,005 und 6 Gew.-%, bezogen auf das Gesamtgewicht der Zusammensetzung, vorliegen.

16. Zusammensetzung nach einem der Ansprüche 7 bis 15, die mindestens ein Oxidationsmittel umfasst, das aus Wasserstoffperoxid, Harnstoffperoxid, Alkalimetallbromaten, Persalzen, Persäuren und Oxidase-Enzymen und vorzugsweise Wasserstoffperoxid ausgewählt ist.

17. Zusammensetzung nach einem der Ansprüche 7 bis 16, die mindestens ein hydroxylhaltiges Lösungsmittel, wie Ethanol, Propylenglykol, Glycerin, Monoetherpolyole, umfasst.

18. Zusammensetzung nach einem der Ansprüche 7 bis 17, die mindestens einen Hilfsstoff umfasst, der aus anionischen, kationischen, nichtionischen, amphoteren, zwitterionischen oberflächenaktiven Mitteln oder ihren Gemischen, anionischen, kationischen, nichtionischen, amphoteren, zwitterionischen Polymeren oder ihren Gemischen, mineralischen oder organischen Verdickungsmitteln und insbesondere assoziativen anionischen, kationischen, nichtionischen und amphoteren Verdickungsmitteln, Antioxidantien, Durchdringungsmitteln, Sequestrierungsmitteln, Parfums, Puffern, Dispersionsmitteln, Konditionierungsmitteln, wie flüchtigen oder nicht flüchtigen, modifizierten oder nicht modifizierten Silikonen, filmbildenden Mitteln, Ceramiden, Konservierungsmitteln, Trübungsmitteln ausgewählt ist.

19. Verfahren zum Färben von Keratinfasern, wobei man die Kosmetikzusammensetzung nach einem der Ansprüche 7 bis 18 auf die Keratinfasern aufbringt, die Zusammensetzung für einen Zeitraum zwischen 3 Minuten und 1 Stunde und vorzugsweise zwischen 15 Minuten und 45 Minuten ruhen lässt und anschließend die Fasern spült.

20. Verfahren zum Aufhellen von Keratinfasern, wobei man die Kosmetikzusammensetzung nach einem der Ansprüche 7 bis 18 ohne Oxidationsmittel und Oxidationsbase auf die Keratinfasern aufbringt, gleichzeitig mit der oder anschließend an die Färbezusammensetzung eine Oxidationszusammensetzung aufbringt, die Zusammensetzungen für einen Zeitraum zwischen 3 Minuten und 1 Stunde und vorzugsweise zwischen 15 Minuten und 45 Minuten ruhen lässt und anschließend die Fasern spült, mit Shampoo wäscht, erneut spült und trocknet.

21. Verfahren zum Färben von Keratinfasern, wobei man die Kosmetikzusammensetzung nach einem der Ansprüche 7 bis 18, die mindestens eine Vorstufe eines Oxidationsfarbstoffs und kein Oxidationsmittel umfasst, auf die Keratinfasern aufbringt, gleichzeitig mit der oder anschließend an die Färbezusammensetzung eine Oxidationszusammensetzung aufbringt, die Zusammensetzungen für einen Zeitraum zwischen 3 Minuten und 1 Stunde und vorzugsweise zwischen 15 Minuten und 45 Minuten ruhen lässt und anschließend die Fasern spült, mit Shampoo wäscht, erneut spült und trocknet.

22. Färbevorrichtung mit mehreren Unterteilungen oder Färbe-"Kit", **dadurch gekennzeichnet, dass** es eine erste Unterteilung, die eine Zusammensetzung nach einem der Ansprüche 7 bis 18 ohne Oxidationsmittel und Oxidationsbase enthält, und eine zweite Unterteilung, die eine ein Oxidationsmittel umfassende Zusammensetzung enthält, umfasst.

23. Vorrichtung nach Anspruch 22, **dadurch gekennzeichnet, dass** die erste Unterteilung eine Vorstufe eines Oxidationsfarbstoffs enthält.

24. Verwendung einer Zusammensetzung nach einem der Ansprüche 7 bis 18 zum Färben von Keratinfasern.

25. Wässrige Färbezusammensetzung zum Färben von Keratinfasern, insbesondere menschlichen Keratinfasern, wie Haaren, die in einem geeigneten Medium Folgendes umfasst:
- mindestens eine Leukoverbindung der Formel (Ib), die eine Vorstufe der Verbindung der Formel (Ia) nach einem der Ansprüche 1 bis 6 ist 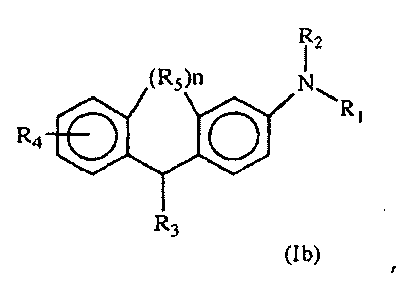
worin R₁, R₂, R₃, R₄, R₅, R₆, R₇ und n die gleiche Bedeutung wie in dem Triarylmethanderivat-Direktfarbstoff der Formel (Ia) haben,
- mindestens eine Oxidationsbase und/oder
- mindestens einen anderen Direktfarbstoff als diejenigen der Formel (Ia).

26. Verfahren zum Färben von Keratinfasern, wobei man die Kosmetikzusammensetzung nach Anspruch 25 ohne Oxidationsmittel auf die Keratinfasern aufbringt, gleichzeitig mit der oder anschließend an die Färbezusammensetzung eine Oxidationszusammensetzung aufbringt, die Zusammensetzungen für einen Zeitraum zwischen 3 Minuten und 1 Stunde und vorzugsweise zwischen 15 Minuten und 45 Minuten ruhen lässt und anschließend die Fasern spült, mit Shampoo wäscht, erneut spült und trocknet.

27. Färbevorrichtung mit mehreren Unterteilungen oder Färbe-"Kit", **dadurch gekennzeichnet, dass** es eine erste Unterteilung, die eine Zusammensetzung nach Anspruch 25 ohne Oxidationsmittel enthält, und eine zweite Unterteilung, die eine ein Oxidationsmittel umfassende Zusammensetzung enthält, umfasst.
